# EUROPEAN PATENT SPECIFICATION

(11) **EP 0 740 156 B1**
(45) Date of publication and mention of the grant of the patent: **19.09.2001**
(21) Application number: 96302858.4
(22) Date of filing: 24.04.1996
(51) Int. Cl.: G01N 33/543, C12Q 1/68

(54) **Immuno and enzyme assays based on surface second harmonic and sumfrequency generation**
Immuno- und Enzymassays basierend auf zweiter harmonischer und Summenfrequenzerzeugung auf einer Oberfläche
Essais immunologiques et enzymatiques basés sur une surface générant une seconde harmonique et une fréquence somme

(30) Priority: 27.04.1995 GB 9508559
(43) Date of publication of application: 30.10.1996
(73) Proprietor: POLYTECHNOLOGY TRANSFER LIMITED, London W11 2HR (GB)
(72) Inventor: Quinn, Peter J., Wheatley OX33 1UH (GB); McStay, Daniel, Petercalter, Aberdeen (GB); Yang, Liqun, Aberdeen AB2 1XG (GB)

(56) References cited:
- EP-A- 0 175 585
- DATABASE WPI Section Ch, Week 9006 Derwent Publications Ltd., London, GB; Class B04, AN 90-039569 XP002009683 & JP-A-01 314 949 (CANON KK) , 20 December 1989
- Phys. Rev. A, vol.28, pages 1883-1885, (1983)

## Description

### BACKGROUND OF THE INVENTION

### 1. Field of the Invention.

The detection and measurement of a large number of compounds within complex biological and biochemical material is a basic requirement of clinical and forensic laboratories and is increasingly important in environmental monitoring and the biotechnology industry. This invention relates to the use of nonlinear optical methods of surface second-harmonic and sum-frequency generation to detect and quantify antibody-antigen interactions, polynucleotide hybridisation and enzyme-substrate complexes.

### 2. Description of the Prior Art.

The basic principal that underlies nearly all quantitative immunoassays is the addition of a reagent to a test sample which results in the formation of a complex of the reagent and a specific component within the sample. The concentration of the reagent which remains unbound compared to that which is bound provides a measure of the specific component. In many (heterogeneous) tests it is necessary to separate the unbound from bound reagent. Usually this is done by some physical process which is often time consuming, expensive and wasteful of manpower and materials. In other tests where secondary procedures are employed to visualise the formation of complexes, there is often a long incubation period before the reaction can be detected. Other problems with conventional immunoassays include the expense, short useful half-life and toxicity of the reagents. For example, one of the most sensitive and widely used assay techniques is radioimmuncassay. However, this technique suffers from both the health hazard posed by the radioactive reagent and waste products, as well as a useful lifetime which is limited by the half-life of the radioactive label.

Two strategies are commonly employed in non-radioactive detection (1) direct methods where enzymes or fluorphores are incorporated into the probe (2) indirect where probes are modified by ligands (biotins/haptens) and detected after by conjugates of ligand-specific proteins with lanthanide or enzymes. Optical detection methods include absorbance and fluorescence measurements.

Methods which do not require separation of free and bound reagent are known and employ the unique optical features presented at interfaces between media. One method that exploits this feature is surface plasmon resonance; this has been commercialized to detect biospecific reactions (BIAcore: Pharmacia® , Sweden). Surface light-bending and evanescent waveguide immunosensor techniques have also been described (U.S. Pat. 4880 752). There are no reports or patents describing the use of nonlinear optical signals for bioassays.

However, Phys. Rev. A, vol. 28, pages 1883-1885, (1983) describe the measurement of the coverage of a quartz surface by p-nitrolsenzoic acid by surface second hormonic generation methods.

### SUMMARY OF THE INVENTION

The present invention consists of a method for immunoassay, polynucleotide hybridization and enzyme assay as defined in claim 1 that exploits changes in the properties of the medium in the interfacial region resulting from the binding reaction that can be detected by nonlinear optical techniques.

The essential features of the invention are:
1. The utilisation of a nonlinear optical process to directly quantitate an interaction between biological molecules (antibodies, antigens, polynucleotides, enzymes, enzyme substrates and analogues).
2. The detection is surface specific so that the assay can be performed in the presence of unreacted components/species that do not interact with the surface layer. This eliminates the need to separate the bound and unbound components.

### DESCRIPTION OF THE PREFERRED EMBODYMENTS

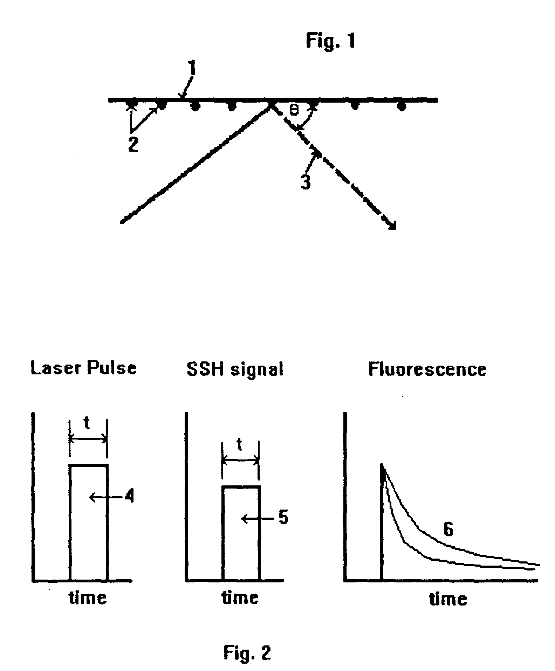

Fig. 1 shows that second-order optical processes such as sum- and difference-frequency generation are forbidden in media with inversion symmetry but such symmetry will necessarily be broken at an interface, where the nonlinear processes are thus allowed. Second-order optical processes originate from the field and structural discontinuity at the interface. Therefore alterations in the surface **1** e.g. by the formation of a surface molecular layer **2** leads to a measurable change in the detected surface second-harmonic signal **3.** The signals are also strongly dependent upon the angle Θ of excitation-emission vector with respect to the surface and degree of order of molecular orientation. Therefore, if the orientation or surface nonlinear coefficient of a surface layer is altered e.g. by the formation of a complex between the surface molecules and a second molecule the characteristics of the second-harmonic signal (e.g. polarisation, magnitude and direction) will also be altered.

The surface nonlinear susceptibility will exhibit a resonant enhancement when the output frequency of the laser for 2f is close to a molecular excitation of a surface species (antigen, antibody, polynucleotide, enzyme, enzyme substrate or substrate analogue). This may be achieved by use of a suitable laser or a tuneable laser or alternatively by covalent or other attachment of a reporter molecule, which possess a molecular excitation close to for 2f, to the antigen, antibody, polynucleotide, enzyme, enzyme substrate or substrate analogue thereby producing a condition of resonance enhancement.

A simple extension of the present invention occurs when the light incident upon the interface contains two frequencies f1 and f2 (e.g. when two lasers are employed) then f1 = f2 may no longer be true. In this case the output is no longer at 2f but at f1 + f2 such a case constitutes sum-frequency generation. This is still a second-order nonlinear optical effect which is surface specific and shares the other properties of surface second-harmonic, as described above. In addition, if the antigen, antibody, polynucleotide, enzyme, enzyme substrate or substrate analogue or a ligand attached to any of these above mentioned molecules has a distinct electronic or vibrational transition, surface sum-frequency generation can be used to selectively detect and quantitate the presence the molecule possessing the particular transition at the surface or changes in the molecule, when located at the surface, which affect the said transition. This may be achieved by ensuring that at least one of the output frequencies of the source corresponds to the target transition. The same effect can be achieved by using a tuneable source. Surface sum-frequency generation, according to an application of the invention, can be used as a basis of a non-separation surface-based bioassays as described in this specification.

Enhancement of the nonlinear optical signals can be achieved by:
1. Attachment of ligands to any of the interacting species that serve to increase the structural and/or electric field discontinuity at the surface.
2. Attachment of a charged particle to any of the interacting species that change the surface electric field on complex formation.
3. Attachment of ligands which possess magnetic properties to any of the interacting species which alter the magnetic field at the interface on complex formation.
4. Attachment of ligands with a large hyperpolarizibility to any of the interacting species whose orientation is altered by complex formation.
5. Attachment of charged or magnetic species or groups with a large hyperpolarizability to the surface whose properties are altered by complex formation so as to modulate the nonlinear optical signal.

Refinement of the nonlinear optical signal (increase in signal to noise ratio) can be achieved as described in Fig 2. The signal **5** has the exact temporal profile as the laser excitation pulse **4** and can be easily time gated whereas fluorescence life-time **6** depends on local environment.

A number of distinct advantages of a surface sensing scheme based on surface second-harmonic generation over existing optical assay schemes are obvious.
1. The surface second-harmonic signal is up-shifted in frequency. It is therefore well removed from potential optical noise sources such as fluorescence and phosphorescence of the target species and background luminescence associated with components in biological material, which are all down-shifted in frequency. Interference from the excitation source is also minimised.
2. The large up-shift in the frequency makes filtering the signal against unwanted background light straightforward.
3. Accurate time-gating of the signal, eg. using a boxcar, can reduce spurious signals by direct comparison with the excitation pulse thus improving the signal to noise ratio of the assay.
4. Being a laser-induced process it is coherent and highly directional.
5. Modulation of the nonlinear optical signal by application of external electric or magnetic fields, especially when charged or magnetic materials are located in the surface, can be used to discriminate the signal.
6. The sensing scheme offers potential reductions in time, cost (per test) and skilled manpower requirements over assay systems presently in use.

In the invention antibodies, antigens, polynucleotides or enzymes are attached to the sensor surface. In one embodyment of the invention multiple tests may be performed where the surface comprises spacially-separated surface domains to which different antibodies and/or antigens and/or polynucleotides and/or enzymes and/or enzyme substrates and/or enzyme inhibitors are attached. The surface is then brought into contact with a solution which may contain the complementary species ie antigen, antibody, polynucleotide or enzyme inhibitor. Formation of a complex between the complementary species will result in a modification of the surface nonlinear optical properties. Measurement of the magnitude, angular dependence or any other parameter dependent on changes of nonlinear optical properties such as surface second-harmonic generation can be used to determine the amount of complex formation at the surface.

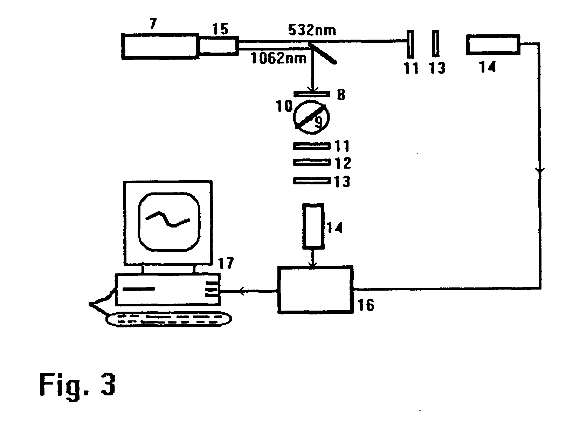

A schematic diagram for detection of nonlinear optical signals is shown in Fig. 3. The beam of a Q-switched Nd:YAG laser **7** is directed through a polariser **8** onto the sample **9** mounted on a calibrated rotation stage **10.** The nonlinear optical signal passes through a neutral density filter **11,** a 1062nm blocking filter **12** and a 532nm band pass interference filter **13** and is detected by a photomultiplier tube **14**. The detector response is fed, together with that recorded from a frequency doubled beam produced by a frequency doubler **15**, into a boxcar **16** and the output dumped to a computer **17** for processing and display.

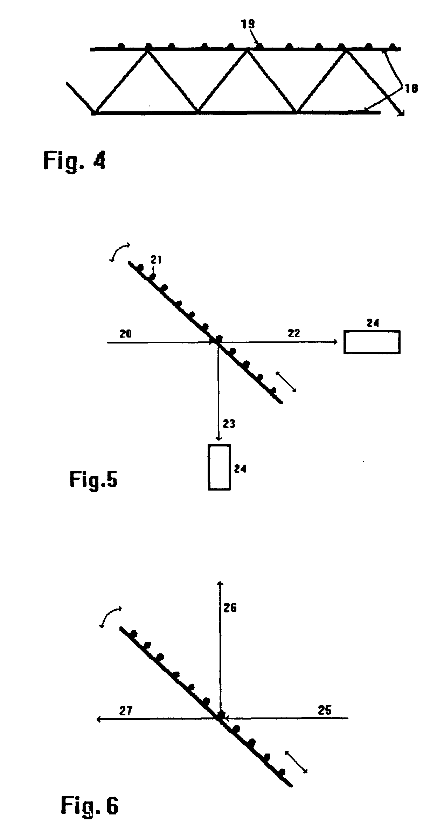

Different formats for interrogating surfaces are illustrated in Figs. 4-6. Fig. 4 shows a waveguide **18** in a total internal reflection format with the biomolecular layer **19** located at the surface. A direct surface probe format is shown in Fig. 5 which is suitable for transparent surfaces consisting of glasses or preferably polymer plastics in which there is greater scope for mutually orienting molecules on the surface. The incident light beam **20,** after interacting with the biomolecular layer **21,** is transmitted **22** and reflected **23** and its intensity recorded using detectors **24.** Signal selectivity can be enhanced using both transmitted and reflected beams by exploiting its angular dependence. The format shown in Fig. 6 is suitable for metal surfaces and other nontransparent surfaces since the incident light beam **25** can interact with the biomolecular layer and the reflected beam **26** can be detected: the transmitted beam **27** can be detected if the material is transparent. Measurements can be performed after removal of the sensor surface from contact with the solution containing unbound reagents (dip-stick format) or while in contact with the solution.

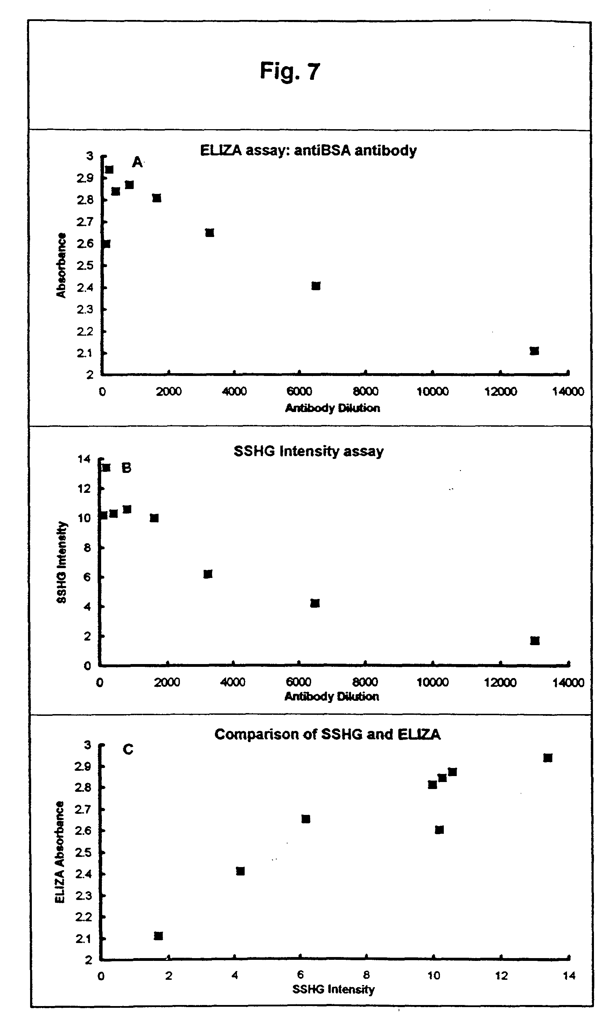

Glass, polymer and metal surfaces are found to exhibit strong surface second-harmonic signals which are modulated in proportion to the amount of randomly-oriented biomolecules adsorbed onto the surface. A comparison of a conventional ELISA assay of bovine serum albumin (BSA) using anti-BSA antibody/HRP-linked anti-IgG antiserum with the surface second-harmonic generation method is shown in Fig. 7. Fig. 7A is the result obtained for dilution of antibody in the ELISA assay performed in a standard Nunc® plastic assay tray. Fig. 7B is the nonlinear optical signal intensity obtained at 45° to the incident beam recorded from the underside of the same wells shown in Fig. 7A. Similar results were obtained from samples containing only BSA and anti-BSA antibody. Fig. 7C is a comparison of the two methods.

The invention can be used for all types of immunoassay including those involving immunoglobulins, specific receptor proteins, hormones, drugs and like compounds and all enzymes for which an inhibitor is known. Enzyme assays in one aspect of the invention are configured by the use of specific inhibitors which bind to the catalytic site according to well characterised association constants (competitive inhibitors). Inhibitors are known for most enzymes that are closely related structurally to the substrate but are not reactive. These inhibitors compete with the substrate for binding to the catalytic site of the enzyme. In one aspect of the invention the change in the surface second harmonic signal produced by binding of the substrate to the surface bound enzyme is used to determine the substrate concentration. In another aspect of the invention inhibitor may be modified or labeled to enhance the field or structural discontinuity at the surface produced by the binding of the inhibitor to the enzyme. Such a technique could be used for continuous monitoring/assays.

Polynucleotide hybridisation can be detected by changes in nonlinear optical signals which occur when polynucleotide in solution hybridizes to a complementary polynucleotide attached to the surface.

## Claims

1. A non-separation method of assay in which analytes being antibodies or antigens or golynucleotides or enzymes or enzyme inhibitors in a soluble phase are contacted with a surface, to which reactants being specific antibodies or antigens or polynucleotides or enzymes or enzyme substrates or enzyme inhibitors are localized, and the change in nonlinear optical properties caused by the specific interactions between analytes and reactants is detected and quantitated by surface second-harmonic generation or sum-frequency generation.

2. A method described in 1 where the analyte is present in a soluble phase that flows over the sensor surface.

3. A method as described in 1 or 2 where the surface comprises a glass, polymer or metal material with reactants being attached or adsorbed to the surface and where interaction with analytes in the soluble phase alters the nonlinear optical properties of the surface layer.

4. A method as claimed in 1 where assay of polynucleotide hybridization is enhanced by inclusion of antibody specific for double-stranded polynucleotide in the soluble phase.

5. A method as described in 1 or 2 where the change in nonlinear optical properties on complex formation is enhanced by attaching a ligand to any of the interacting species that will increase a structural and/or electric field discontinuity at the surface on complex formation.

6. A method described in 1 or 2 in which the nonlinear optical properties of the surface is modulated by application of external electric fields.

7. A method as described in 1 or 2 where complex formation is performed so as to cause the molecules of the interacting species to be aligned in a preferred orientation on the sensor surface.

## Patentansprüche

1. Eine nicht-trennende Essay-Methode bei der die Agentien Antikörper oder Antigene oder Polynukleotide oder Enzyme oder Enzyminhibitoren in löslicher Phase mit einer Oberfläche in Kontakt gebracht werden, an welcher die Reagentien spezifische Antikörper oder Antigene oder Polynukleotide oder Enzyme oder Enzymsubstrate oder Enzyminhibitoren lokalisiert sind. Die Änderung von nichtlinearen optischen Eigenschaften hervorgerufen durch spezifische Wechselwirkungen von Agentien und Reagentien wird nachgewiesen und durch die Erzeugung der zweiten Harmonischen an Oberflächen (surface second-harmonic) oder von Summenfrequenzen quantifiziert.

2. Eine Methode wie in 1) beschrieben, bei der das Agens in einer löslichen Phase vorliegt die über die Sensor-Oberfläche fließt.

3. Eine Methode wie in 1) oder 2) beschrieben bei der die Oberfläche ein Glas, Polymer oder Metall darstellt und die Reagentien an die Oberfläche gebunden oder adsorbiert sind und bei der eine Wechselwirkung mit Agentien in der löslichen Phase die nichtlinearen optischen Eigenschaften der Oberflächenschicht ändert.

4. Eine Methode wie in 1) beschrieben bei der ein Polynukleotid-Hybridisierungs-Assay durch den Einschluss von für doppelsträngige Polynukleotide in löslicher Phase spezifische Antikörper verstärkt wird.

5. Eine Methode wie in 1) oder 2) beschrieben bei der Änderungen der nichtlinearen optischen Eigenschaften bei Komplexbildung dadurch verstärkt werden daß ein Ligand an ein beliebiges der wechselwirkenden Spezies bindet und dadurch strukturelle Inhomogenitäten oder Inhomogenitäten des elektrischen Feldes bei Komplexbildung vergrößert.

6. Eine Methode wie in 1) oder 2) beschrieben bei der nichtlineare optische Eigenschaften der Oberfläche durch die Anwendung von externen elektrischen Feldern moduliert werden.

7. Eine Methode wie in 1) oder 2) beschrieben bei der eine Komplexbildung derart durchgeführt wird daß eine bevorzugte Orientierung der wechselwirkenden Spezies auf der Sensoroberfläche hervorgerufen wird.

## Revendications

1. Une méthode de dosage par un changement des propriétés optiques non linéaires d'une surface est décrite, méthode qui ne nécessite pas la séparation physique de la forme libre et liée des réactifs ou des analytes, tels que anticorps, antigènes, polynucléotides, enzymes, substrats ou inhibiteurs d'enzymes, et ceci après un contact où les réactifs solubles sur une surface où les antigènes, anticorps, polynucléotides complémentaires, inhibiteurs ou substrats ou enzymes se sont liés de manière spécifique. Les changements des propriétés optiques non linéaires de la surface sont détectés et quantifiés par la génération sur la surface d'un signal de seconde harmonique et la génération par sommation de fréquences.

2. Une méthode de dosage dérivée de la méthode décrite en 1. où la forme soluble est entraînée en regard de la surface sensible par un flux liquide ou gazeux.

3. Une méthode de dosage dérivée des méthodes décrites en 1 et en 2 où la surface sensible est constituée de verre, de polymères plastiques ou de métal et sur laquelle les réactifs sont liés ou adsorbés, l'interaction des réactifs avec les analytes de la phase volumique allant modifier les propriétés optiques non linéaires de la surface.

4. Une méthode dérivée de la méthode décrite en 1. où le dosage de l'hybridation entre les enchaînements polynucléotidiques est facilité par la présence dans la phase volumique d'un anticorps reconnaissant les arrangements en double-brins.

5. Une méthode de dosage dérivée des méthodes décrites en 1 et en 2. où le changement des propriétés optiques non linéaires est augmenté du fait de la liaison d'un ligand ou d'un groupement chimique sur une des espèces moléculaires en interaction, ceci dans le but d'accroître la discontinuité de champs électrique et de structure représentée par l'interface.

6. Une méthode de dosage dérivée des méthodes décrites en 1 et en 2. où le changement des propriétés optiques non linéaires de la surface est modulé par application d'un champs électrique externe.

7. Une méthode de dosage dérivée des méthodes décrites en 1 et en 2. où la formation du complexe spécifique est réalisée de telle sorte que les espèces moléculaires en interaction soient alignées dans une orientation particulière par rapport à la surface.
